# EUROPEAN PATENT SPECIFICATION

(11) **EP 2 461 854 B1**
(45) Date of publication and mention of the grant of the patent: **01.05.2013**
(21) Application number: 10740383.4
(22) Date of filing: 19.07.2010
(51) Int. Cl.: A61M 5/44, F28F 3/12, F28F 21/06, F28F 3/04

(54) **HEAT EXCHANGE CIRCUIT**
WÄRMETAUSCHERKREIS
CIRCUIT D'ÉCHANGE DE CHALEUR

(30) Priority: 03.08.2009 IT TV20090156
(43) Date of publication of application: 13.06.2012
(73) Proprietor: SIS-TER S.p.A., 26020 Palazzo Pignano (Cremona) (IT)
(72) Inventor: FINI, Massimo, I-26020 Palazzo Pignano (CR) (IT)
(74) Representative: Belloni, Giancarlo
(86) International application number: PCT/IB2010/053279
(87) International publication number: WO 2011/015961

(56) References cited:
- EP-A2- 0 397 487
- WO-A1-2008/063121
- GB-A- 2 451 113
- US-A1- 2002 116 041
- US-A1- 2006 211 986

## Description

The invention relates to a circuit for heat exchange to/from a liquid, in particular for heat exchange between a device and a liquid or between two liquids. Even more particularly, the invention relates to a disposable circuit for heat exchange to/from a physiological liquid. The invention relates moreover to the thermal device suitable for use together with the disposable circuit.

During therapeutic treatment which requires the infusion of liquids into a patient and/or extracorporeal circulation it is required to monitor constantly the temperature of the fluid which is infused into the patient.

In some cases it is necessary for the liquid to be heated before infusion, in order to avoid the risk of inducing an undesirable state of hypothermia in the patient. In such cases, the temperature of the liquid, which is typically blood or an infusion liquid, must be as close as possible to the physiological body temperature of the patient.

In other cases, such as for example certain surgical operations, a controlled state of hypothermia must be induced in the patient. By artificially lowering the body temperature of the patient it is in fact possible to slow down the metabolic processes of the organism so as to reduce the oxygen intake. In this way, it is possible to suspend circulation of the blood for relatively long periods of time and thereby prevent the lack of blood flow and consequent hypoxia from causing damage to the tissues.

For example, intense hypothermia, achieved by lowering the patient's temperature to about 20° C, allows complete stoppage of the blood circulation for periods of about 30 minutes. In the case, instead, of extracorporeal circulation, usually hypothermia of average intensity (about 28 to 32° C) is induced to ensure optimum protection of the tissues, in particular the brain tissues.

The hypothermia is induced by externally cooling the patient, but in particular cooling the blood using special heat exchangers able to extract heat from the blood. At the end of the operation it ic obviously required to raise the patient's temperature back to the physiological values; heating of the blood is usually performed using the same extracorporeal circuit, but with the heat being transferred to the blood, instead of being extracted from it.

Also, in haemodialysis treatment, heating of the dialysis solution is required essentially in all cases since, with this operation, the extracorporeal blood circulation can be kept at standard temperature values.

Heating the blood is necessary in the event of major transfusions and/or in cases where the transfusion is not programmed and there is not enough time to prepare the bags and heat them to room temperature.

When heating the blood it must also be remembered that an excessively high temperature may be extremely dangerous for the patient. For example, at a temperature of 41 ° C the brain tissues start to suffer damage, while a temperature of 45° C is almost certainly lethal. For this reason very strict monitoring of the circuit for heating the infusion liquids is required.

In any case, both in the case where the physiological liquid to be infused must be heated and in the case where it must be cooled, certain intrinsic difficulties associated with this particular area of application must be resolved. The heat exchange circuits of the known type, although widely used, are not without defects.

The main defect of the known circuits is the general low level of heat exchange efficiency. Heat exchange must be performed in real time along the line used to convey the physiological liquid. Poor efficiency means slowing down of the infusion operation and/or the need to ensure that greater quantities of liquid are present inside the exchanger (priming).

Where there is inefficient heat exchange, it is also required to use a notable temperature gradient in order to speed up the transfer of heat to/from the liquid. This may result, for example in the case where the blood must be heated, in the presence of a means which is able to perform heating to a temperature well above 45° C and is therefore extremely dangerous. In fact, it may happen that the speed of infusion decreases and, consequently, the blood remains inside the heat exchange circuit for longer than programmed. In such a case, too high a temperature of the heating means could also easily raise the temperature of the blood to a dangerous level. It can therefore be understood how a heat exchange circuit of this type is intrinsically dangerous for heating blood and therefore requires an extremely sophisticated system of sensors.

Another drawback of the circuits of the known type is associated with the cost, in particular in the case where they are circuits of the disposable type.

The object of the present invention is therefore to solve at least partially the drawbacks identified in connection with the heat exchange circuits of the known type.

The aim of the present invention is to provide a heat exchange circuit which has a high efficiency.

Moreover, the aim of the present invention is to provide a heat exchange circuit which has a simple design and low cost.

An example of prior art is given in US-2002/116041.

The abovementioned aims and object are achieved by a heat exchange circuit according to Claim 1.

The characteristic features and further advantages of the invention will emerge from the description provided hereinbelow, of a number of examples of embodiment, provided purely by way of a non-limiting example, with reference to the accompanying drawings in which:
Figure 1 shows schematically two walls suitable for forming a heat exchange circuit not according to the invention;
Figure 2 shows schematically the two walls according to Figure 1 in a different configuration;
Figure 3 shows schematically a side view of a heat exchange circuit not according to the invention;
Figure 4.a shows schematically the pattern in the first wall of a heat exchange circuit not according to the invention;

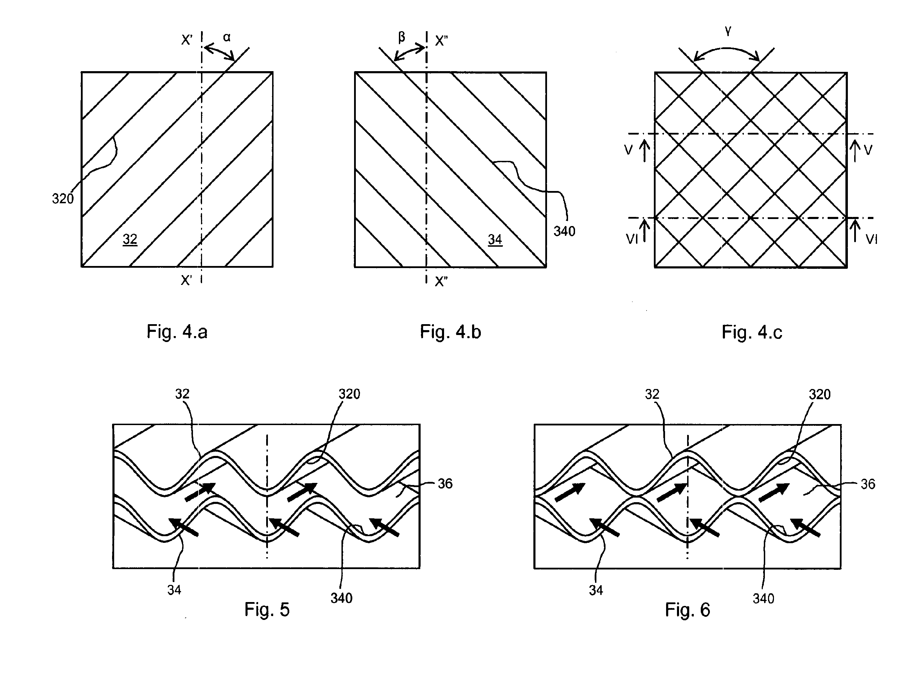

Figure 4.b shows schematically the pattern of a second wall of a heat exchange circuit not according to the invention;
Figure 4.c shows schematically the superimposed arrangement of the patterns according to Figures 4.a and 4.b;
Figure 5 shows a heat exchange circuit according to the invention, viewed in cross-section along the line V-V of Figure 4.c;
Figure 6 shows a heat exchange circuit according to the invention, viewed in cross-section along the line VI-VI of Figure 4.c;

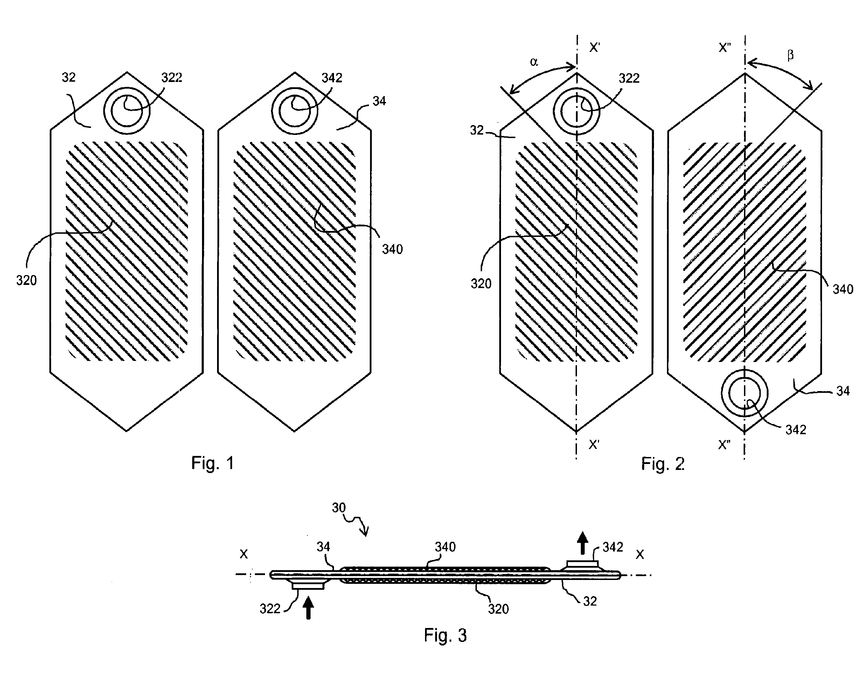

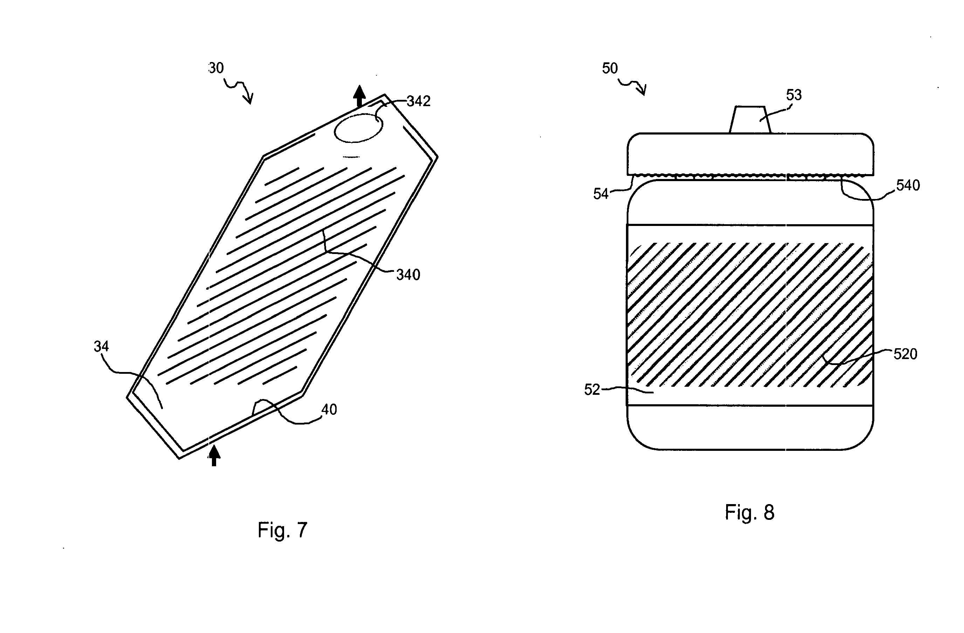

Figure 7 shows schematically a perspective view of a heat exchange circuit not according to the invention, similar to that of Figure 3;
Figure 8 shows schematically a plan view of the thermal device not according to the invention, with the lid raised;

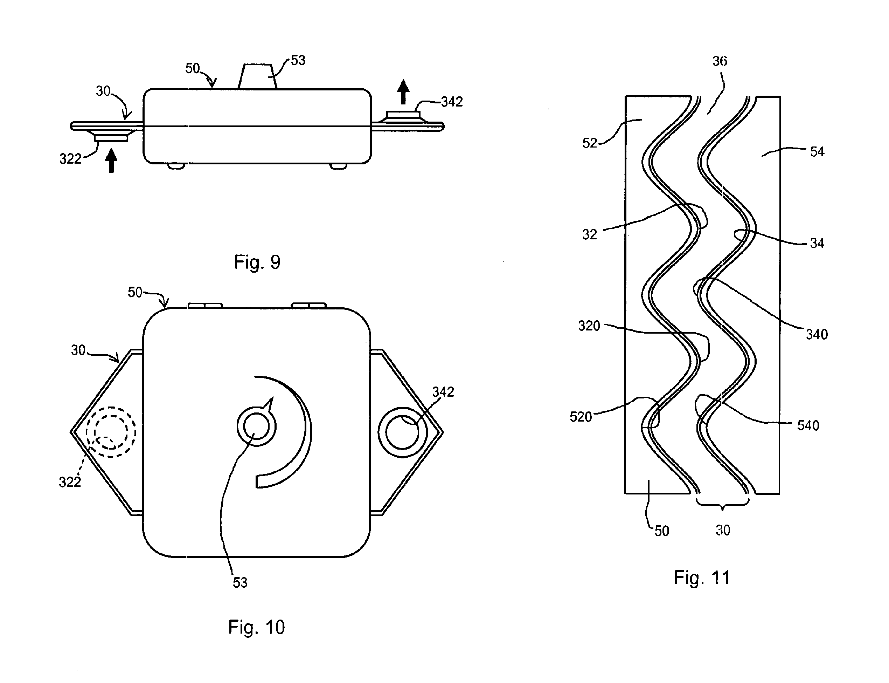

Figure 9 shows schematically a plan view of an assembly consisting of a heat exchange circuit of the type shown in Figure 3 and the thermal device according to Figure 8;
Figure 10 shows schematically a side view of the assembly according to Figure 9;
Figure 11 shows schematically a cross-sectional view of the assembly according to Figure 9;

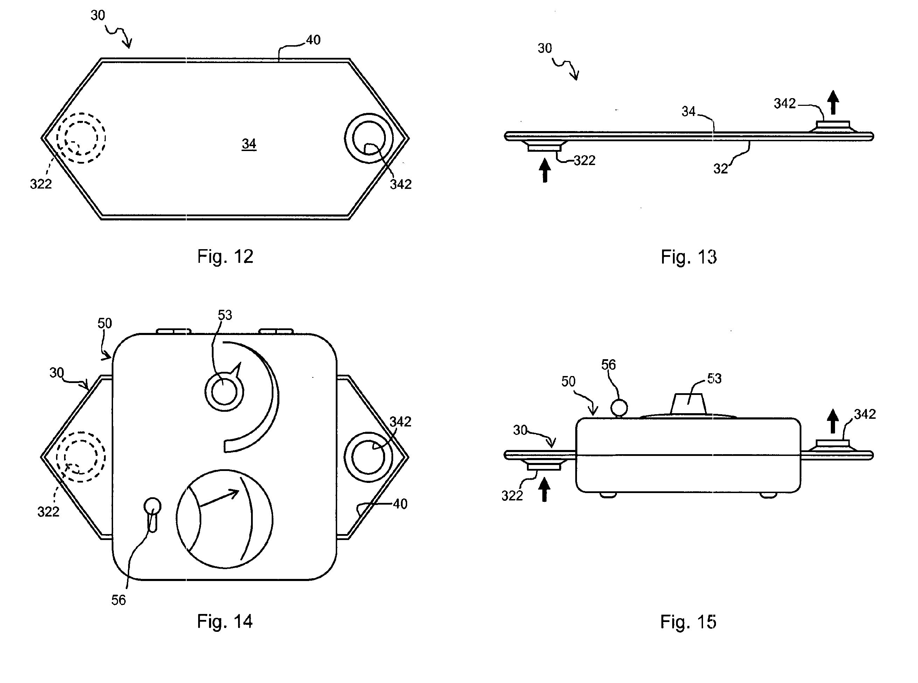

Figure 12 shows schematically a plan view of a heat exchange circuit not according to the invention;
Figure 13 shows schematically a side view of the heat exchange circuit according to Figure 12;
Figure 14 shows schematically a plan view of an assembly consisting of a heat exchange circuit of the type shown in Figure 12 and a thermal device not according to the invention;
Figure 15 shows schematically a side view of the assembly according to Figure 14;

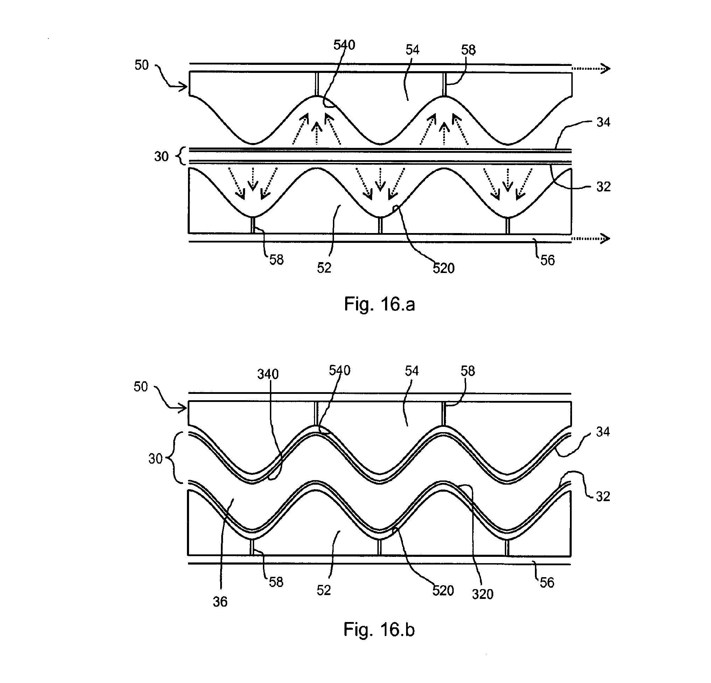

Figure 16 shows schematically a cross-sectional view of the assembly according to Figure 14 in two different configurations;

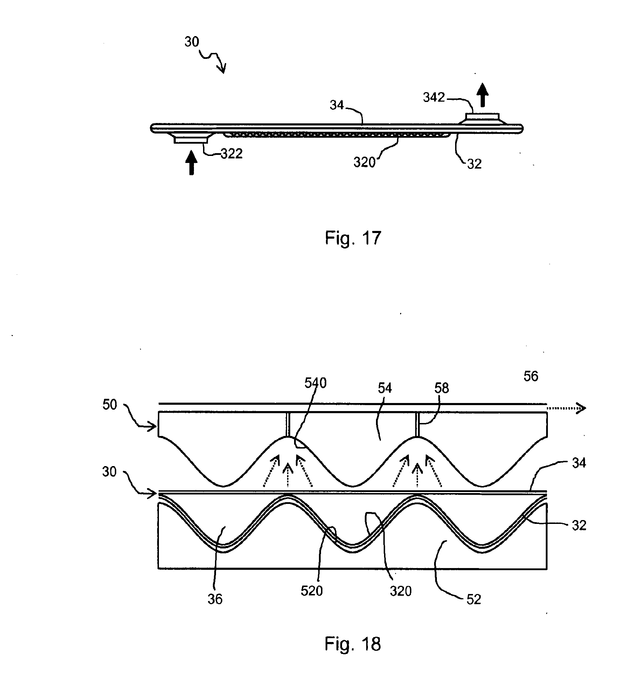

Figure 17 shows schematically a side view of a heat exchange circuit not according to the invention;
Figure 18 shows schematically a cross-sectional view of an assembly consisting of a heat exchange circuit of the type shown in Figure 17 and a thermal device not according to the invention;

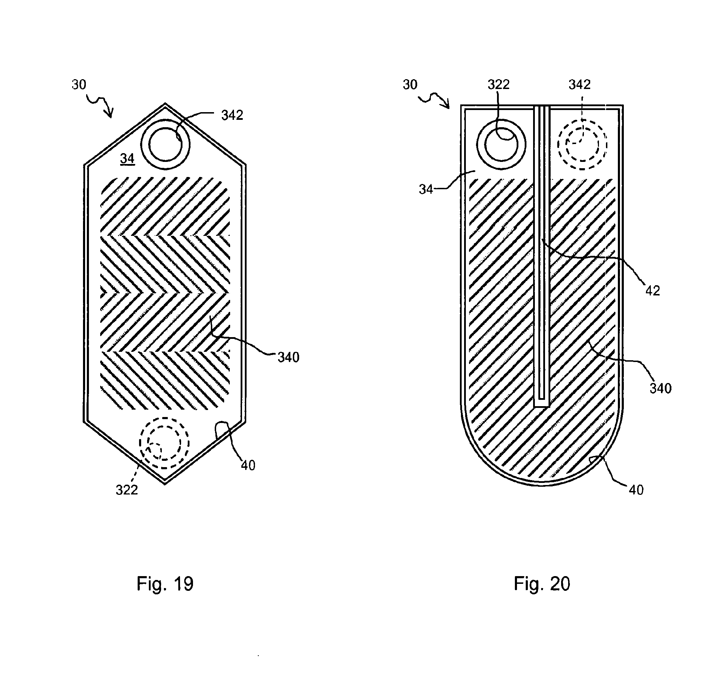

Figure 19 shows schematically a plan view of a heat exchange circuit not according to the invention;
Figure 20 shows schematically a plan view of a heat exchange circuit not according to the invention;

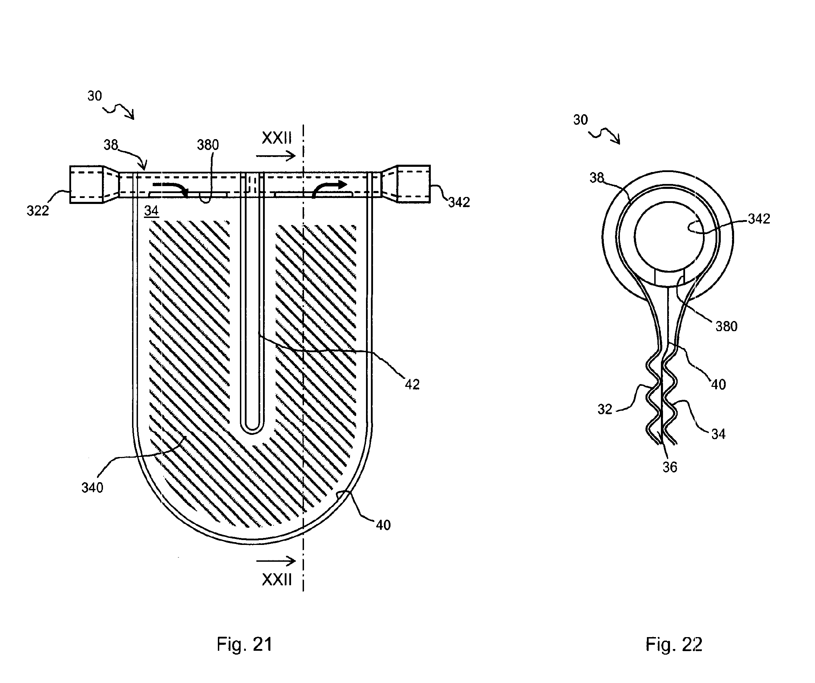

Figure 21 shows schematically a plan view of a heat exchange circuit not according to the invention;
Figure 22 shows schematically an enlarged detail of the cross-section along the line XXII-XXII in Figure 21;

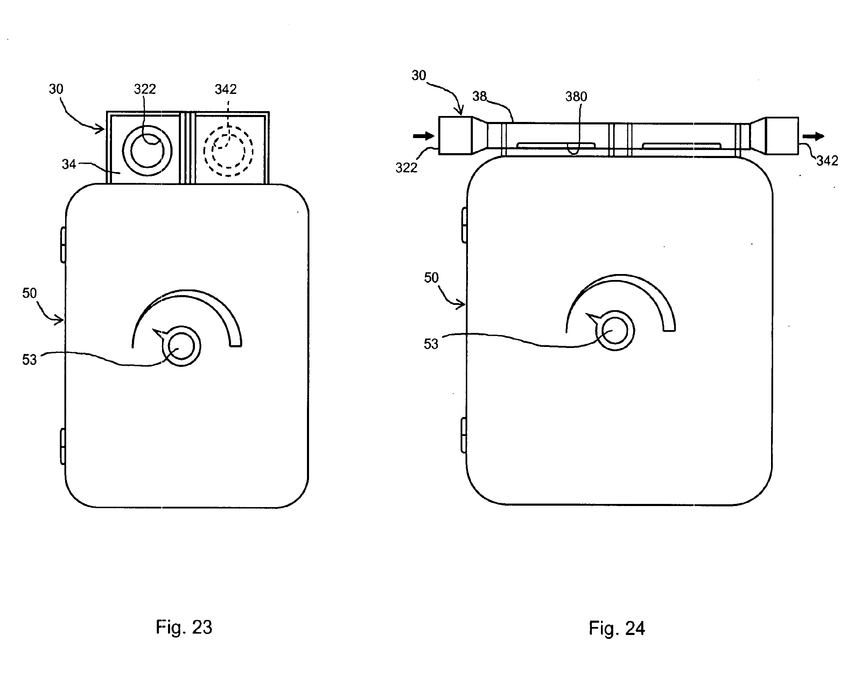

Figure 23 shows schematically a plan view of an assembly consisting of a heat exchange circuit of the type shown in Figure 20 and a thermal device not according to the invention;
Figure 24 shows schematically a plan view of an assembly consisting of a heat exchange circuit of the type shown in Figure 21 and a thermal device not according to the invention;

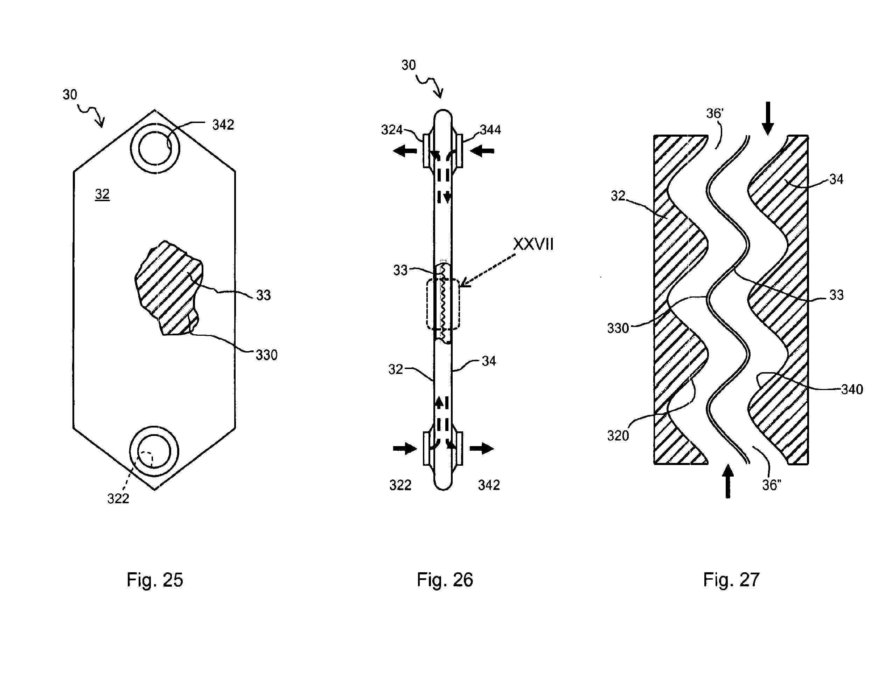

Figure 25 shows schematically a plan view of a heat exchange circuit according to the invention, partially cut-away;
Figure 26 shows schematically a side view of the heat exchange circuit according to Figure 25, partially cut-away;
Figure 27 shows the device indicated by XXVII in Figure 26;

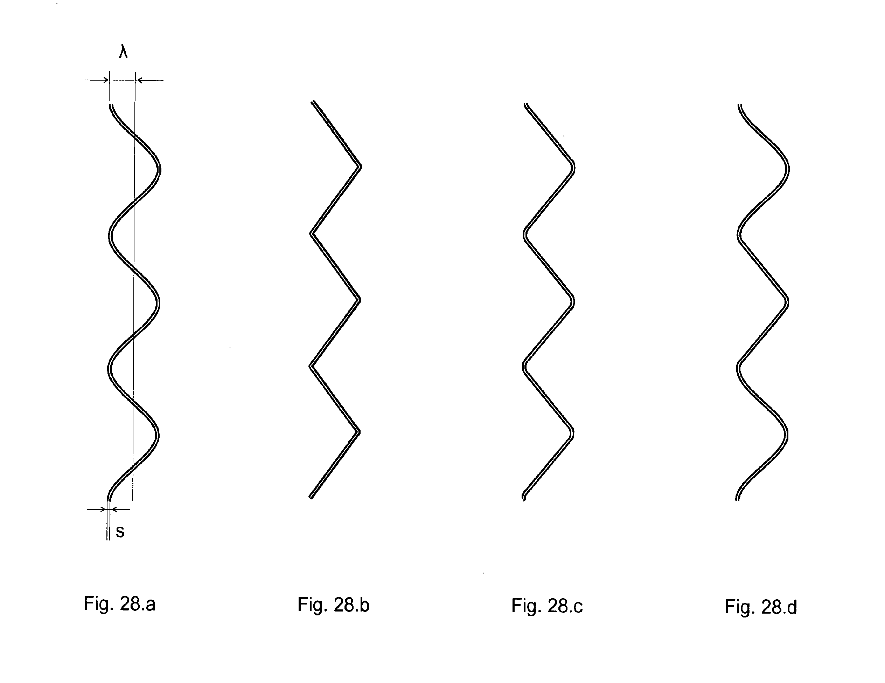

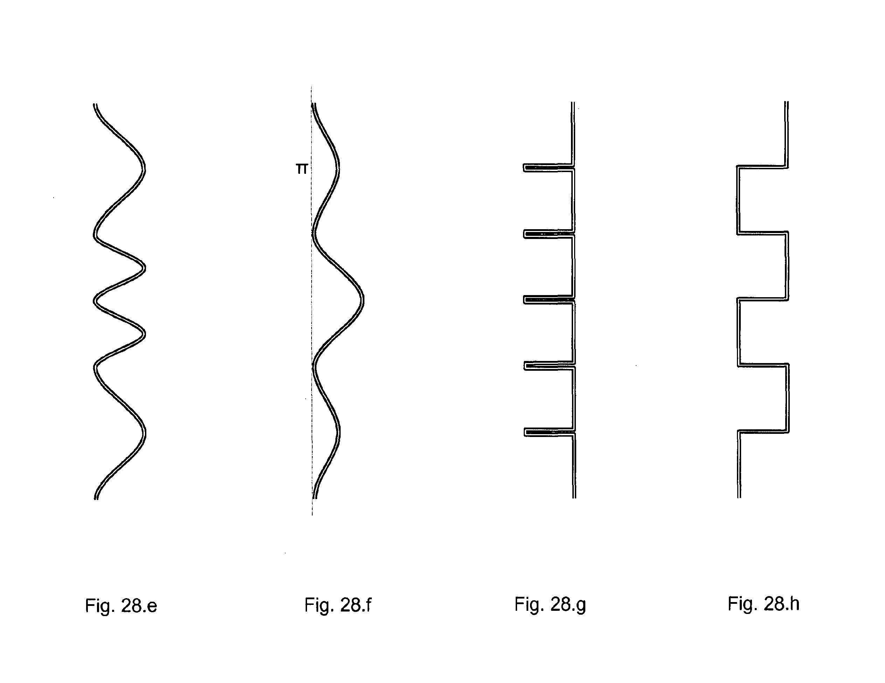

Figure 28 shows some possible configurations of the corrugations of the circuit according to the invention;

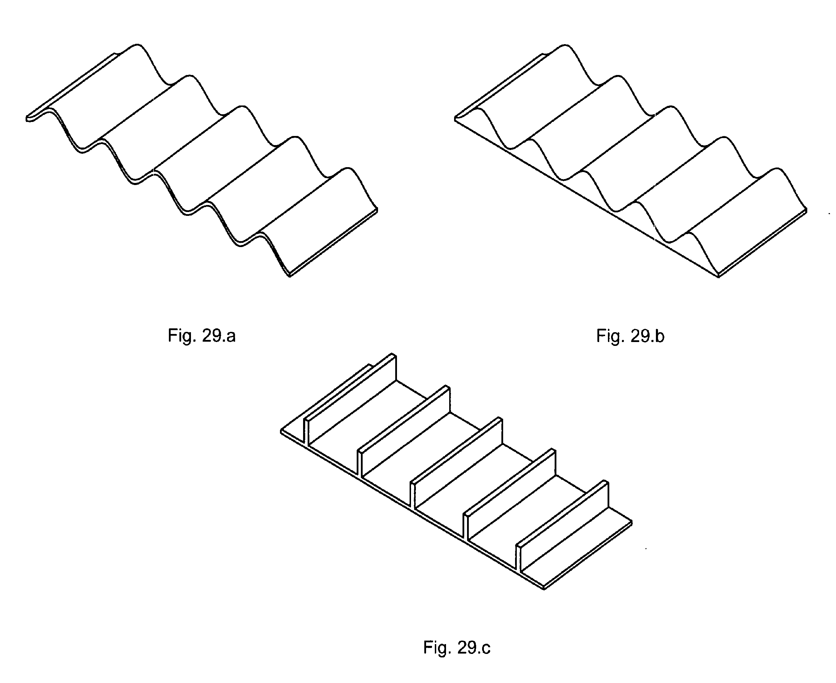

Figure 29.a shows a portion of a first type of wall of a heat exchange circuit according to the invention;
Figure 29.b shows a portion of a second type of wall of a heat exchange circuit according to the invention;
Figure 29.c shows a portion of the third type of wall of a heat exchange circuit according to the invention;

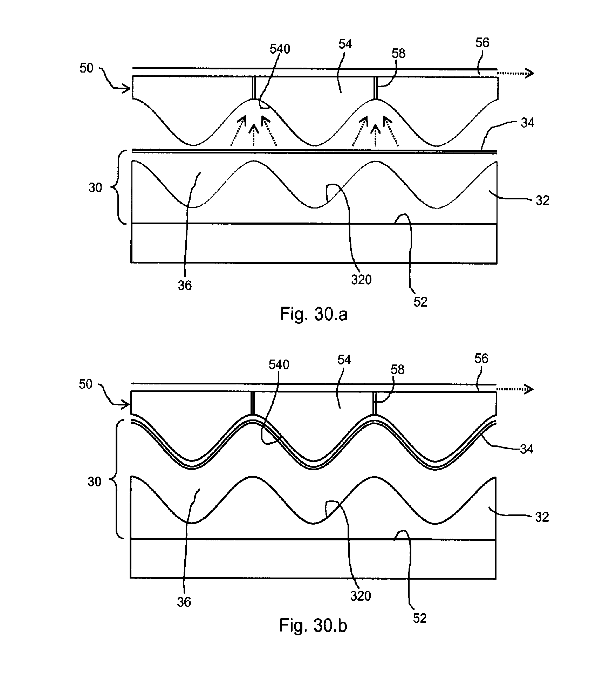

Figures 30 show schematically a cross-sectional view of an assembly consisting of a heat exchange circuit and a thermal device according to the invention in two different configurations;

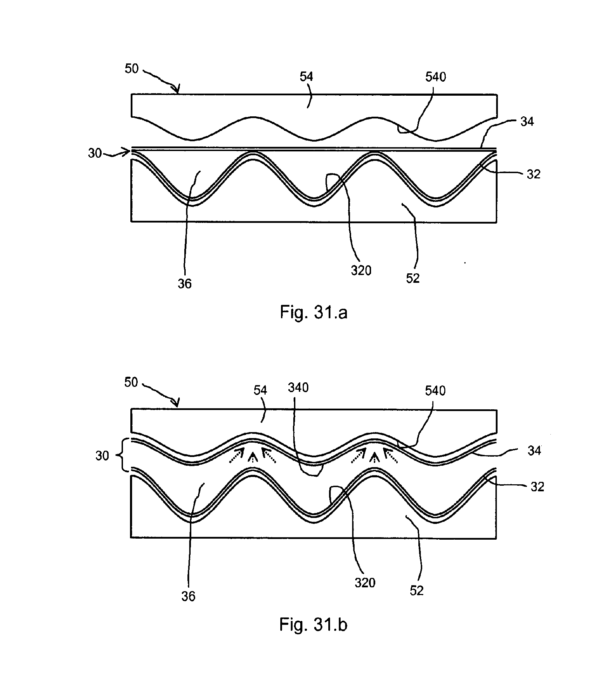

Figures 31 show schematically a cross-sectional view of an assembly consisting of a heat exchange circuit and a thermal device according to the invention in two different configurations.

The present invention relates to a heat exchange circuit, indicated in its entirety by 30. The circuit 30 is designed to promote heat exchange to/from a physiological fluid. According to an illustrative embodiment, a heat exchange circuit comprises at least two walls 32 and 34 which rest against each other and are sealed so as to form a duct 36 able to contain a flow of physiological fluid. Each one of the at least two walls 32 and 34 comprises, during use, a pattern of corrugations 320 and 340. The corrugations have, at least along sections, a preferential direction. The pattern of corrugations 320 of one wall 32 is positioned so as to be superimposed at least partially on the pattern of corrugations 340 of the other wall so as to form an angle γ between the respective preferential directions, the angle γ ranging between 70° and 110°. Finally, at least one of the walls 32 or 34 is designed to transfer heat to/from the liquid flow.

Reference will be made below, in relation to the heat exchange circuit 30, to the concepts "inner" and "outer". "Inner" is understood as referring to the parts of the circuit which, during use, are wetted by the liquid; on the other hand, "outer" refers to the parts of the circuit which, during use, are not wetted by the liquid.

By way of example, the circuit 30 according to the illustrative embodiment shown in Figures 1 to 11, is now described in detail. In particular, Figures 1 and 2 show the two walls 32 and 34 before they are joined together to form the circuit 30. Figure 1 shows the two walls 32 and 34 next to each other and arranged with the same orientation. As can be noted, they are entirely identical, even though this is not strictly necessary; each of them comprises a pattern of corrugations, 320 and 340, respectively, and an opening 322 and 342, respectively. According to this embodiment, the walls 32 and 34 have a small thickness in relation to the amplitude of the corrugations. This means that the same pattern of corrugations appears on both sides of the wall.

In the schematic illustration shown in the accompanying figures, the patterns of corrugations, for example 320 and 340, are shown as hatched areas on the surfaces of the walls. The lines of the hatching may be regarded as being a schematic representation of the peaks of the corrugations or, equally well, a schematic representation of the valleys of the corrugations. Figure 2 shows the two walls 32 and 34 arranged next to each other. The first wall 32 has retained the same orientation, while the second wall 34 has been rotated twice with respect to the orientation shown in Figure 1. Said second wall has undergone a first rotation through 180° in its plane and a second rotation through 180° about its longitudinal axis X". It can be noted how the pattern of corrugations 340 of the wall 34 is perfectly visible also on the opposite side, owing to the fact that, in the present embodiment, the wall 34 has a small thickness in relation to the amplitude of the corrugations.

From this configuration, with a simple translational movement, the two walls 32 and 34 may be arranged on top of each other so that the two corrugation patterns 320 and 340 coincide. In the example described here the walls are formed so that, machining tolerances excluded, the two corrugation patterns are superimposed perfectly on top of each other. Figure 2 shows for each one of the two walls 32 and 34 preferential directions of the corrugations. More specifically, the pattern 320 has a preferential direction which forms an angle α with the longitudinal axis X' of the wall 32, while the pattern 340 has a preferential direction which forms an angle β with the longitudinal axis X" of the wall 34. Obviously, in the light of that described above, α and β are the same angle conventionally attributed an opposite sign. However, in other embodiments, there is nothing to prevent α and β being different from each other in terms of absolute values.

In the embodiments described here, the amplitude of α and β is substantially equal to 45°. This value represents an excellent compromise between conflicting design requirements: on the one hand it is opportune to limit the head losses in the circuit 30, while on the other hand it is required to ensure a high degree of mixing inside the fluid. The first requirement would lead to a reduction in the amplitude of the angles α and β to about 30°, while the second requirement would increase it to about 60°. In practice, specific tests carried out by the Applicant have shown how angles α and β with an amplitude of between 35° and 55° ensure limited head losses and optimum mixing.

Obviously, as the person skilled in the art may easily understand from the above description, the angle γ formed between the two preferential directions of the two corrugation patterns is defined by the sum of the amplitudes α and β. In the specific case, therefore, the angle γ has an amplitude equal to about 90°, while in other embodiments of the invention it may vary between 70° and 110°.

Figure 3 shows a side view of a circuit 30 according to an illustrative embodiment. It is obtained by superimposing the walls 32 and 34 (in the configuration shown in Figure 2) and welding them along the periphery 40. In Figure 3 it can also be seen how the longitudinal axis X' of the wall 32 and the longitudinal axis X" of the wall 34 are superimposed on each other to form the longitudinal axis X of the circuit 30.

Figure 7 shows a perspective view of a illustrative circuit 30 similar to that shown in Figure 3.

According to the embodiments shown in Figures 3 and 7, the circuit 30 comprises two openings 322 and 342. Here and below the opening 322 will be regarded as an inlet and the opening 342 as an outlet. Obviously nothing would change if the functions of the two openings were assumed to be reversed.

Figure 4 shows in a very simplified form the preferential directions of the corrugations of the two walls considered individually (Figures 4.a and 4.b) and the two walls superimposed (Figure 4.c). This illustration shows furthermore the relationship between the angles α, β and γ. Moreover, Figure 4.c shows by way of explanation two different cross-sectional lines. The line VI-VI divides the grid pattern of corrugations along a contact line between the respective peaks, while the line V-V divides the grid pattern along a line where there is no contact. The cross-sections obtained along these lines are shown schematically in the following Figures 5 and 6. These figures are shown in perspective so as to illustrate more clearly the layout of the network of channels formed by the combination of the two corrugation patterns 320 and 340. Figure 8 shows a thermal device according to an illustrative embodiment, denoted in its entirety by 50. The thermal device 50 is designed to cooperate with the heat exchange circuit 30 and comprises two plates 52 and 54, at least one of which is thermally active. The plates 52 and 54 are shaped so as to ensure intimate contact with the walls 32 and 34 of the circuit 30 at least in the zones coinciding with the respective corrugation patterns 320 and 340.

In the specific embodiment in Figure 8, the thermal device 50 comprises a base, comprising the plate 52, and a lid which can be opened and closed onto the base and which comprises the plate 54. Both the plates 52 and 54 are thermally active. A circuit of the type shown in Figure 7 may be arranged between the lid and the base. Figures 9 and 10 show the assembly consisting of the heat exchange circuit 30 according to Figure 7 inserted inside the thermal device according to Figure 8. As can be noted, the inlet 322 and the outlet 342 of the circuit 30 inserted inside the thermal device 50 are perfectly accessible and able to be used. In other words, the liquid may be supplied to the circuit 30 and may be drawn off after heat exchange without the positioning of the circuit 30 inside the thermal device 50 having any effect on the circulation of the liquid.

With the lid open as shown in Figure 8 it is possible to note how the plate 52 comprises a pattern of corrugations 520 identical to the pattern 320 of the wall 32 with which it is intended to come into contact. As a result it is possible to ensure intimate contact between the wall 32 of the circuit 30 and the thermally active plate 52. The same intimate contact is similarly achieved also between the wall 34 and the plate 54.

The intimate contact between the two corrugation patterns is illustrated in the schematic cross-section shown in Figure 11. This cross-section and all the following cross-sections have been simplified for greater clarity. The cross-section in Figure 11 does not show the perspective layout of the network of channels formed by the corrugations; for this layout reference should be made again to Figures 5 and 6.

Figure 11 shows, instead, the intimate contact between the patterns, i.e. shows how the corrugations of the plates 52 and 54 match perfectly the outer corrugations of the walls 32 and 34. In particular it can be seen how the peaks of the corrugations of the plates 52 and 54 fit perfectly inside the peaks of the corrugations of the walls 32 and 34, respectively, and also how the valleys of the corrugations in the plates 52 and 54 fit perfectly inside the valleys of the corrugations in the walls 32 and 34, respectively.

The intimate contact between the corrugation patterns ensures that the walls 32 and 34 rest fully on the thermally active plates 52 and 54, so as to prevent the presence of air between the walls and the plates. Air is in fact not a good heat conductor and its presence between the walls and the plates would adversely affect the heat exchange efficiency.

In Figures 11, 16, 18, 30 and 31, for greater clarity, the walls 32 and 34 are shown slightly spaced from the respective plates 52 and 54, but in reality this spacing is not present.

At least one of the plates 52 and 54 is defined here as being thermally active. It is in fact able to assume a temperature different from the external temperature of the walls of the circuit 30. More specifically, according to certain embodiments, the thermally active plate 52 or 54 may assume higher temperatures so as to release heat to the walls of the circuit 30. According to other embodiments, the thermally active plate 52 or 54 may assume lower temperatures so as to absorb heat from the walls of the circuit 30. According to further other embodiments, the thermally active plate 52 or 54 may assume higher or alternatively lower temperatures so as to release or alternatively absorb heat to/from the walls of the circuit 30. The temperature of the thermally active plate 52 or 54 can be preferably regulated via suitable regulating means 53. According to some embodiments, only one of the two plates 52 and 54 is thermally active, while in other embodiments both of them are active.

The plates 52 and/or 54, depending on the different embodiments of the thermal device 50, may comprise different means for rendering them thermally active. These means may consist of electric resistors which are designed to raise the temperature, or Peltier cells, which are able both to raise the temperature and, by reversing the polarity of the power supply, to lower it.

The walls 32 and 34, according to the embodiment described hitherto, may be made of polymer material or metallic material. These materials must be suitable for retaining the form imparted to them during production, especially as regards the corrugations. A stable form of the corrugations in fact ensures intimate contact between the walls of the circuit 30 and the plates of the thermal device 50. If, however, the corrugations should be deformed, this intimate contact would be affected, air would enter between the walls, and the plates and the heat exchange efficiency would be drastically reduced.

In the case where polymer material is used, the walls must have a particularly small thickness in order to compensate for the fairly low thermal conductivity which is typical of the polymers. Obviously, from among the polymers suitable for contact with physiological liquids, all other conditions being equal, it is preferable to choose a polymer with a relatively high thermal conductivity. According to certain embodiments, the thermal conductivity of the polymer may be increased by means of the addition of suitable fillers dispersed in the polymer itself. These fillers may be, for example, in a manner known per, metal and/or ceramic particles. In the case where a metallic material is used for the walls 32 and 34, this may be chosen, for example, from aluminium, titanium and stainless steel. Moreover, the walls made of metallic material could if necessary comprise a thin polymer lining should it be required to improve the biocompatibility thereof or other desirable characteristics such as the resistance to corrosion and/or to aggressive chemical agents.

With particular reference to Figure 28.a, the thickness s of the polymer walls advantageously ranges between 30 and 300 µm, while the thickness of the metal walls advantageously ranges between 100 and 200 µm. The thickness of the corrugations (or wave amplitude λ) advantageously ranges between 500 and 3000 µm.

Figures 12 to 16 show a different embodiment of the heat exchange circuit 30 and, consequently, the associated thermal device 50.

The heat exchange circuit 30 shown in Figures 12 and 13 comprises two smooth walls 32 and 34, i.e. without the corrugation patterns described above. With the sole exception of the corrugations not present, the heat exchange circuit 30 has a form similar to that of the preceding figures. The thermal device 50 according to Figures 14 to 16 is similar to that described above, but comprises plates which, in addition to being thermally active, also have a suction function. As can be noted in the schematic cross-section in Figure 16.a, the thermal device encloses internally the circuit 30 with the smooth walls, as shown in Figures 12 and 13. The plates 52 and 54 of the thermal device 50 comprise corrugation patterns 520 and 540, respectively, which are substantially similar to those described above. According to this embodiment, however, the plates 52 and 54 also comprise suction conduits 58 which are connected to the vacuum application means 56 of the thermal device 50. By means of this configuration it is possible to suck off the air trapped between the smooth walls 32 and 34 and the corrugated plates 52 and 54. As a result of the air suction, which is schematically indicated by the dotted-line arrows in Figure 16.a, the walls 32 and 34 may be made to adhere fully against the plates 52 and 54 so as to reproduce exactly the corrugations thereof The final configuration of the walls 32 and 34 in intimate contact with the plates 52 and 54 is schematically shown in Figure 16.b. In the same way as already indicated for Figure 11, in Figure 16.b also, the walls 32 and 34 are slightly spaced from the plates 52 and 54 for greater clarity, but in reality this spacing is not present and on the contrary contact is forcibly provided by means of the action of the vacuum.

For this purpose, the suction conduits 58 are preferably arranged in arrays on the bottom of the valleys of the corrugations of the plates 52 and 54. This arrangement of the suction conduits 58 is shown in Figure 16.

According to other embodiments of the thermal device 50, the plates 52 and 54 are made of porous material which is obtained for example by means of sintering of metal powders of suitable particle size. The porous plates 52 and 54 allow widespread suction of the air trapped between the walls 32 and 34 and therefore without the presence of the suction conduits 58.

According to this embodiment of the heat exchange circuit, the walls 32 and 34 must necessarily be deformable so as to be able to reproduce accurately, during use, the corrugation patterns of the suction plates 52 and 54.

In the case where the circuit 30 is made of polymer material, deformation of the walls 32 and 34 may to a large extent consist of an elastic deformation. Therefore, when the vacuum is no longer applied, the corrugations become almost entirely flat. In the case where the circuit 30 is instead made of metallic material or a different polymer material, deformation of the walls 32 and 34 to a large extent consists of a plastic deformation. Therefore, even when the vacuum is no longer applied, the corrugations remain substantially unaffected.

The embodiment according to Figures 17 and 18 is a mixed form where the wall 32 is preformed so as to comprise the patterns of corrugations 320 as well as the walls of the circuit 30 shown in Figures 1 to 11. In contrast, the wall 34 is smooth and must be corrugated at the time of use, as must be the walls of the circuit 30 according to Figures 12 to 16. Figure 18 shows schematically a cross-sectional view of the assembly consisting of the circuit 30 according to Figure 17 and the respective thermal device 50. In particular the thermal device 50 comprises a plate 52 similar to that of the device 50 according to Figures 8 to 11 and the other plate 54 similar to the device 50 according to Figures 14 to 16.

According to this embodiment of the heat exchange circuit 30, the walls 32 and 34 may be made of materials different from each other.

Figure 19 shows a heat exchange circuit 30 similar to that of Figures 1 to 11 in which, however, the preferential direction of the corrugations may be defined solely along sections within the patterns 320 and 340. In this embodiment, in particular, the corrugations define four different homogeneous areas, i.e. each with a well defined preferential direction.

According to other possible embodiments it is possible for the homogeneous areas to be present in a different number and/or arrangement. It has been noted, during specific tests carried out by the Applicant, that the transition between two adjacent homogeneous areas, with a consequent change in preferential direction, results in substantial advantages in terms of mixing inside the flow.

Figure 20 shows a different embodiment of the circuit 30 according to the invention. According to this embodiment, the two walls 32 and 34 are sealed with respect to each other not only along the periphery, but also partially along the centre line 42. In this way, therefore, the circuit 30 has a substantially U-shaped extension in which the inlet 322 and the outlet 342 are adjacent to each other.

Figure 21 shows an embodiment which is similar to that of Figure 20, but in which the U-shaped circuit 30 also comprises a top pipe 38 which is relatively rigid with respect to the walls 32 and 34. The top pipe 38 defines the inlet 322 and the outlet 342 of the U-shaped circuit 30. The internal channel of the top pipe 38 is interrupted so that the liquid is forced to pass through the side openings 380 which allow it to flow into and out of the duct 36. Figure 22 shows in detail the cross-section of the circuit 30 according to Figure 21 at the point where the top pipe 38 is joined to the walls 32 and 34.

Figures 20 and 21 show both the U-shaped 30 circuits in embodiments in which the walls comprise preformed corrugations. This illustration makes it easier to understand how the corrugations may be arranged and how they are able to define a common preferential direction along the entire U-shaped circuit. With this U-shaped configuration, in fact, the axis X of the entire circuit, relative to which the angles α and β of the preferential directions of the corrugations are defined, is not readily definable. The possibility of determining the angle γ, defined by the preferential directions of the two superimposed patterns of corrugations, obviously remains unchanged.

It is perfectly possible, in other embodiments, for the abovementioned U-shaped circuits to be formed with smooth walls, such as those described in relation to the circuit 30 of Figures 13 to 16, or with mixed walls, such as those described in relation to the circuit 30 according to Figures 17 and 18.

Figure 23 and 24 shows two assemblies according to another illustrative embodiment. They comprise U-shaped circuits, according to the embodiments shown in Figures 20 and 21, respectively, and the corresponding thermal devices 50 associated with them.

Obviously, in the case where the U-shaped heat exchange circuits 30 are of the smooth-wall or mixed-wall type, the respective thermal devices 50 must also comprise the vacuum application means 56 such as those described above in connection with Figures 14 to 16 and 18.

Figures 25 to 27 show the heat exchange circuit according to the invention. According to the invention, the circuit comprises three walls 32, 33 and 34 so as to define two different ducts 36' and 36" which are separate from each other. The outer walls 32 and 34 of the circuit 30 each define two openings. It is thus possible to define an inlet 322 and an outlet 324 for the duct 36' and an inlet 344 and an outlet 342 for the duct 36". This type of heat exchange circuit 30 is not intended to co-operate with a thermal device 50, but is instead intended to be used as an exchanger of heat between two different liquid flows at different temperatures.

The heat exchange circuit 30 according to Figures 25 to 27 also comprises corrugation patterns similar to those of the other embodiments described above. The corrugation patterns in this case are three in number, one for each wall 32, 33 and 34.

As can be seen in Figure 27, the outer walls 32 and 34 have a large thickness in relation to the amplitude of the corrugations. As a result, in this and other cases, the corrugations may be maintained only on the inside of the circuit, while the outer surfaces of the circuit may be smooth.

On the other hand, the inner wall 33 has a small thickness in relation to the amplitude of the corrugations such that the same corrugation pattern appears on both sides of the wall itself.

In this particular embodiment, the outer walls 32 and 34 are intended to prevent as far as possible heat exchange between the inside of the circuit and the surrounding environment. For this reason, the outer walls 32 and 34 have a large thickness and are preferably made of polymer material with a low thermal conductivity coefficient. On the other hand, the inner wall 33 is intended to facilitate as far as possible heat exchange between the two ducts 36' and 36". For this reason, the inner wall 33 has a small thickness and is preferably made of metallic material with a high thermal conductivity coefficient.

Figures 29.a and 29.b show schematically two wall portions, which are respectively thin and thick, with the same type of corrugation. Figures 29.b and 29.c show schematically wall portions, which are both thick, with different types of corrugation. In the description of the heat exchange circuits 30 reference has always been made to walls with a small thickness, with the sole exception of the outer walls 32 and 34 of the circuit shown in Figures 25 to 27.

According to the embodiment of Figures 30, the circuit 30 comprises a thick wall 32 in which the corrugations appear on the inner side only, while the outer side is smooth. In this embodiment, the thick wall is preferably made of metallic material. Indeed, as already mentioned above, metallic materials have high thermal conductivity and thus allow the use of large wall thicknesses. The thermal devices 50 designed to cooperate with these embodiments of the circuit 30 have thermally active plates with a smooth surface. The smooth plates ensure that the desired intimate contact with the externally smooth walls of the circuit 30 is obtained. In the particular embodiment shown in Figures 30, the wall 34 is of the thin and smooth type, but there is nothing to prevent sit from being one of the other types described here.

In the embodiment of Figures 31, the wall 32 is preformed so as to comprise the pattern of corrugations 320, like the walls of the circuit 30 according to Figures 1 to 11. In contrast, the wall 34 is smooth and must be corrugated at the time of use. Differently from that shown in Figures 16, 18 and 30, however, in this case the formation of the corrugations is not performed by the application of the vacuum from the outside. In the embodiment according to Figures 31, the corrugations of the wall 34 are obtained as a result of internal pressure of the fluid which presses the wall 34 against the plate 54. The effect of this pressure is schematically indicated by means of the dotted-line arrows in Figure 31.b. For this reason the figure shows corrugations with a wave amplitude λ which is smaller for the wall 34 than that of the corrugations preformed on the wall 32. According to this embodiment of the heat exchange circuit 30, the walls 32 and 34 may be made of materials and/or with thicknesses which are different from each other.

In the embodiments of the circuit 30 described above, the corrugations have a configuration which is substantially comparable to a sinusoid of given amplitude and wavelength. According to other embodiments, however, the progression may be of a different nature. Some configurations are shown by way of example in Figures 28. Figure 28.a shows the substantially sinusoidal progression of the embodiments described above. Figure 28.b shows a broken-line progression in which straight sections are connected together by sharp angles.

This configuration would be optimal from the point of view of the mixing which could be induced in the flow, but in most cases with the technologies for forming such corrugations it is not possible to obtain a sharp angle. Figure 28.c shows a broken-line configuration in which straight sections are connected together by curved portions. This configuration represents a technologically feasible configuration similar to the previous configuration. This configuration is all the more similar, the smaller the radius of curvature of the connecting curves. Figure 28.d shows a combined sinusoidal/broken line configuration. Figure 28.e shows a configuration in which the wavelength of the corrugations is not constant along the length of the pattern. Figure 28.f shows instead a further configuration in which the amplitude of the corrugations is not constant along the length of the patterns. In these two specific examples the configuration is sinusoidal, but of course a variation of wavelength and/or amplitude may also be obtained for other configurations, for example of the broken line or mixed type. Figure 28.g shows a configuration in which flanges define channels with a quadrangular cross-section, on one side only of the corrugations. This configuration formed with a thin wall is substantially equivalent to the configuration shown in Figure 29.c, formed with a thick wall. Finally, Figure 28.h shows a configuration in which a square wave defines channels with a quadrangular cross-section, on both sides of the corrugations.

As can be noted, all the configurations shown by way of example, including the configuration with a variable wave amplitude, define a plane of peaks (indicated by the line π in Figure 28.f), at least on one side of the pattern. The presence of such a plane is highly advantageous for being able to define a support surface against another wall and for being able to define a circuit according to the invention.

As the person skilled in the art can understand, the various embodiments of the circuit and/or the thermal device according to the invention have been described purely by way of example. In other words the different possibilities described above in terms of materials, overall form, number of homogeneous areas within the corrugation patterns, corrugation configurations, presence of the vacuum application means, etc., can be differently combined with each other also in ways which are not specifically described. For example, it is possible, without departing from the scope of the present invention, to form a U-circuit with a plurality of homogeneous areas within the corrugation patterns, or a suction thermal device with a pattern which has a non-sinusoidal progression.

As the person skilled in the art can understand from the above description, the particular internal configuration of the circuit 30 according to the invention is able to achieve a high heat exchange efficiency. The arrangement of the corrugation patterns on top of each produces a network of channels which form a duct 36 in which the flow of liquid has a minimum thickness. The minimum thickness of the flow allows minimization of the thermal boundary layer effect, this effect resulting in large temperature differences between the layer of liquid directly in contact with the wall and the layers situated within the flow and at a distance from the wall. The thermal boundary layer effect thus results in the need, in low-efficiency circuits of known type, for high wall temperatures (70-80 °C) in order to compensate for the difficulty in supplying heat to the deep layers within the flow. As already mentioned in the introduction, these wall temperatures are inherently dangerous for the patient's health.

Furthermore, the particular configuration of the corrugation patterns and their arrangement on top of each other ensures intense mixing of the liquid which further limits the boundary layer effect. The liquid which is in direct contact with the wall along a duct section, because of the intense mixing, will almost certainly be situated at a distance from the wall along a subsequent duct section. In other words, the high degree of mixing or turbulence phenomena induced in the flow by the particular configuration of the channel network continuously mixes the liquid, preventing the possible formation of large temperature differences within it.

The significant reduction in the boundary layer effect results in a considerable increase in the heat exchange efficiency in the circuit according to the invention compared to known types of circuits. Because of the high heat exchange efficiency it is thus possible to keep the wall temperature within values which are highly acceptable in terms of the patient's safety (40-70°C, depending on the materials used).

Finally, as the person skilled in the art can understand from the above comments, the heat exchange circuit according to the invention is extremely economical from a manufacturing point of view, being particularly suitable for use as a disposable insert. As can be understood from the above description, many embodiments of the circuit 30 consist three walls 32, 33 and 34. In this respect production of the circuit 30 is greatly simplified since it is sufficient to perform pressing of a large of number of walls of a single type, with obvious advantages in logistics terms.

The invention according to Figures 25 to 27 benefits from this feature. The outer walls 32 and 34 are in fact identical to each other, while the single inner wall 33 requires a different production method.

With regard to the embodiments of the circuit and the thermal device described above, the person skilled in the art may, in order to satisfy specific requirements, make modifications and/or replace elements described with equivalent elements, without thereby departing from the scope of the accompanying claims.

## Claims

1. Circuit (30) for heat exchange to/from a physiological fluid, comprising three walls (32, 33, 34) which rest on each other and are sealed so as to form two ducts (36', 36") separate from each other and suitable for containing two different flows;
wherein each of the three walls (32, 33, 34) comprises, in use, a pattern of corrugations (320, 330, 340) having, at least along sections, a preferential direction, the pattern of corrugations (330) of one wall (33) being positioned so as to be superimposed at least partially on the pattern of corrugations (320, 340) of the other walls (32, 34) so as to form an angle γ between the respective preferential directions, **characterized in that** the angle γ ranges between 70° and 110°;
the outer walls (32, 34) have a large thickness and low thermal conductivity coefficient, and
the inner wall (33) has a small thickness and a high thermal conductivity coefficient and is suitable for transferring heat to/from the fluid flow.

2. Circuit (30) according to Claim 1, wherein at least one wall (33) has a thickness (s) which is small in relation to the amplitude (λ) of the corrugations such that the same pattern of corrugations appears on both sides of the wall (33).

3. Circuit (30) according to Claim 1 or 2, wherein at least one wall (32; 34) has a thickness (s) which is large in relation to the amplitude (λ) of the corrugations such that the pattern of corrugations appears only on the inner side of the wall (32; 34), while the outer side is smooth.

## Patentansprüche

1. Schaltung (30) für einen Wärmeaustausch zu/von einem physiologischen Fluid, mit drei Wänden (32, 33, 34), die aneinander ruhen und so abgedichtet sind, dass zwei Kanäle (36', 36") gebildet sind, die voneinander getrennt und geeignet sind, zwei unterschiedliche Strömungen zu enthalten;
wobei jede der drei Wände (32, 33, 34) im Gebrauch ein Riffelmuster (320, 330, 340) aufweist, das zumindest entlang eines Teils eine Vorzugsrichtung hat, wobei das Riffelmuster (330) von einer Wand (33) so positioniert ist, dass es zumindest teilweise mit dem Riffelmuster (320, 340) der anderen Wände (32, 34) überlagert ist, um so einen Winkel γ zwischen den jeweiligen Vorzugsrichtungen zu bilden, **dadurch gekennzeichnet, dass** der Winkel γ in einem Bereich zwischen 70° und 110° ist;
wobei die äußeren Wände (32, 34) eine große Dicke und einen kleinen Wärmeleitkoeffizienten haben, und
wobei die innere Wand (33) eine kleine Dicke und einen großen Wärmeleitkoeffizienten hat und geeignet ist, Wärme zu/von der Fluidströmung zu übertragen.

2. Schaltung (30) gemäß Anspruch 1, wobei zumindest eine Wand (33) eine Dicke (s) hat, die in Bezug auf die Amplitude (λ) der Riffel derart klein ist, dass dasselbe Riffelmuster an beiden Seiten der Wand (33) erscheint.

3. Schaltung (30) gemäß Anspruch 1 oder 2, wobei zumindest eine Wand (32; 34) eine Dicke (s) hat, die in Bezug auf die Amplitude (λ) der Riffel derart groß ist, dass das Riffelmuster nur an der inneren Seite der Wand (32; 34) erscheint, während die Außenseite glatt ist.

## Revendications

1. Circuit (30) d'échange de chaleur avec un fluide physiologique, comprenant trois parois (32, 33, 34) qui reposent les unes sur les autres et qui sont scellées de manière à former deux conduits (36', 36") séparés l'un de l'autre et appropriés pour contenir deux écoulements différents;
dans lequel chacune des trois parois (32, 33, 34) comprend, en utilisation, un motif d'ondulations (320, 330, 340) ayant, au moins le long de sections, une direction préférentielle, le motif d'ondulations (330) d'une paroi (33) étant positionné de manière à être superposé au moins partiellement au motif d'ondulations (320, 340) des autres parois (32, 34) de manière à former un angle γ entre les directions préférentielles respectives, **caractérisé en ce que** l'angle γ est compris entre 70° et 110° ;
les parois externes (32, 34) ont une grande épaisseur et un faible coefficient de conductivité thermique, et
la paroi interne (33) a une faible épaisseur et un coefficient de conductivité thermique élevé et est appropriée pour transférer la chaleur vers/à partir de l'écoulement de fluide.

2. Circuit (30) selon la revendication 1, dans lequel au moins une paroi (33) a une épaisseur (s) qui est faible par rapport à l'amplitude (λ) des ondulations de sorte que le même motif d'ondulations apparaît des deux côtés de la paroi (33).

3. Circuit (30) selon la revendication 1 ou 2, dans lequel au moins une paroi (32 ; 34) a une épaisseur (s) qui est grande par rapport à l'amplitude (λ) des ondulations de sorte que le motif d'ondulations apparaît uniquement du côté intérieur de la paroi (32 ; 34), tandis que l'autre côté est lisse.
